# FASCICULE DE BREVET EUROPEEN

(11) **EP 1 315 458 B1**
(45) Date de publication et mention de la délivrance du brevet: **01.09.2004**
(21) Numéro de dépôt: 01956642.1
(22) Date de dépôt: 26.07.2001
(51) Int. Cl.: A61B 17/12, A61B 17/00

(54) **DISPOSITIF D'OCCLUSION VASCULAIRE ET APPAREIL D'UTILISATION**
VASKULÄRE OKKLUSIONSVORRICHTUNG, SOWIE GERÄT ZU IHRER VERWENDUNG
VASCULAR OCCLUSION DEVICE AND APPARATUS FOR USING SAME

(30) Priorité: 04.09.2000 FR 0011557
(43) Date de publication de la demande: 04.06.2003
(73) Titulaire: Mialhe, Claude, 83300 Draguignan (FR)
(72) Inventeur: Mialhe, Claude, 83300 Draguignan (FR)
(74) Mandataire: Hautier, Jean-Louis
(86) Numéro de dépôt international: PCT/FR2001/002445
(87) Numéro de publication internationale: WO 2002/019926

(56) Documents cités:
- EP-A- 0 864 300
- WO-A-97/27893
- WO-A-99/07292
- DE-C- 19 801 076
- US-A- 5 382 261

## Description

La présente invention concerne d'abord un dispositif d'occlusion apte à être introduit dans un vaisseau.

Elle concerne également un appareil de mise en place d'un tel dispositif d'occlusion vasculaire .

L'invention trouvera son application dans le domaine de la fabrication et de la mise en oeuvre de prothèses occlusives pour tout type de vaisseau dans le corps humain ou animal.

Des systèmes d'occlusion de vaisseaux existent déjà mais ne donnent pas entière satisfaction.

*On connaît du brevet américain US-A-5.382.261 une méthode et un appareil pour occlure des vaisseaux.*

Selon ce document, une occlusion permanente d'un vaisseau est possible pour une personne par l'utilisation d'un élément flexible d'obstruction fixé à au moins une partie métallique expansible radialement, généralement de forme tubulaire, l'élément flexible d'obturation ayant une configuration longitudinale sensiblement tubulaire.

Selon ce document, l'élément d'occlusion est une sorte de bouchon de forme tubulaire rapporté sur un organe de fixation dans le vaisseau sous forme de stent, composé d'une armature métallique expansible.

Dans cette antériorité, le degré d'occlusion n'est pas réglable.

Il sera généralement total et ce, de façon permanente.

On constate par ailleurs que la fixation du dispositif d'occlusion dans le vaisseau s'effectue par un seul côté de l'élément obturateur.

Les contraintes y sont donc concentrées lorsque la pression sanguine s'applique à l'intérieur du système occlusif.

D'autres systèmes occlusifs ont été proposés notamment celui divulgué dans le document EP-A-0.947.168.

*Y est présenté un dispositif d'occlusion vasculaire autoexpansible revêtu qui comprend un élément sous forme de fil métallique tressé comportant au moins deux éléments de fixation axialement espacé et fixé au fil métallique tressé.*

*Un film fin recouvre au moins la moitié du dispositif.*

*L'élément tressé proposé est apte à s'éteridre radialement lorsqu'il est libéré à l'intérieur du vaisseau sanguin afin d'obturer le passage du sang.*

*Dès sa libération, il réalise immédiatement et complètement l'occlusion du vaisseau*.

Encore une fois, ce système d'occlusion a vocation à occlure de façon permanente et totale le vaisseau.

Sa structure est par ailleurs complexe et présente un coût de fabrication non négligeable.

Par ailleurs, le flux sanguin est orienté vers la périphérie du vaisseau, au niveau de la liaison de l'élément tressé avec les parois internes de ce vaisseau.

Par conséquent, des contraintes importantes y sont engendrées du fait de la pression sanguine qui s'applique.

Pour procurer une étanchéité efficace, il est donc nécessaire de fournir un effort d'expansion important de l'élément tressé sur la paroi interne du vaisseau.

On connaît également du document DE-C-19 801 076 un organe expansible pour l'implantation dans un vaisseau. Selon ce document, l'organe comprend deux zones 16, 18 aptes à être fixées relativement à la paroi du vaisseau pour maintenir l'organe. Une partie intermédiaire creuse est en outre présente pour assurer le passage du flux sanguin. Ce dispositif constitue essentiellement un accessoire pour la réalisation de gestes chirurgicaux aptes à court-circuiter un vaisseau. Il ne s'agit pas d'un dispositif d'occlusion, et aucune déformation par torsion n'est présentée

La présente invention a pour but de pallier les inconvénients des dispositifs connus jusqu'à présent.

Elle permet également d'envisager une pluralité d'applications du dispositif d'occlusion qu'elle présente.

En effet, un premier but de l'invention est de permettre, à volonté, l'obtention d'une occlusion totale ou seulement partielle.

On pourra ainsi adapter le dispositif ici présenté selon la pathologie à traiter.

Une occlusion partielle sera réalisée, par exemple pour le traitement des insuffisances valvulaires veineuses.

D'autre part, une occlusion complète est également possible, par exemple pour le traitement de certains anévrismes artériels.

Elle a l'avantage pour ce faire de réaliser l'occlusion par torsion d'une partie intermédiaire entre deux organes expansibles de fixation sur la paroi du vaisseau.

Il est possible de modifier ou d'ajuster le degré de torsion appliqué à la partie intermédiaire afin de régler le niveau d'occlusion réalisé.

La facilité de réalisation de l'occlusion et de mise en place du dispositif selon l'invention permet aussi d'envisager des occlusions temporaires.

Ainsi, le dispositif pourra être amovible et retiré en fin de traitement.

Une telle possibilité pourra s'appliquer par exemple à la formation de dispositifs anti-emboliques de protection cérébrale lors des angioplasties ou dilatation des carotides, des coronaires ou des pontages coronariens.

Le dispositif occlusif ici présenté a, par ailleurs, l'avantage d'être précis dans le réglage du degré d'occlusion réalisé.

Ce degré d'occlusion peut être ajusté suivant la position angulaire relative des deux organes expansibles ou encore suivant leur écartement longitudinal.

Le mouvement de rotation assure aussi un réglage en longueur in situ du dispositif ainsi que le réglage en position de la zone de striction maximale.

Ce dispositif peut, en outre, être mis en oeuvre avec des moyens de fixation (organes expansibles) d'un type connu et qui ont fait maintenant leur preuve.

Ainsi, les organes expansibles pourront être des endoprothèses auto expansibles à mémoire de forme ou encore elles pourront être positionnées par inflation d'un ballon.

On constate de façon conséquente que l'implantation d'un tel dispositif occlusif est rapide et ne nécessite que des outils parfaitement maîtrisés par le praticien.

Une intervention endoluminale définitive ou non peut ainsi être réalisée en réduisant les temps d'intervention.

Le dispositif peut aussi s'appliquer à l'hémostase des vaisseaux après ponction transpariétale et, plus généralement, à tous types d'occlusion pariétale.

D'autres buts et avantages apparaîtront au cours de la description qui suit qui n'est cependant donnée qu'à titre indicatif et non limitatif de l'invention.

La présente invention concerne un dispositif d'occlusion apte à être introduit dans un vaisseau caractérisé par le fait qu'il comporte deux organes expansibles creux pour sa fixation par appui sur deux portions de la paroi du vaisseau et une partie intermédiaire, creuse, déformable en torsion à un degré ajustable selon la position angulaire relative des deux organes expansibles pour créer une zone de striction maximale définissant le degré d'occlusion.

Ce dispositif pourra se présenter sous les variantes énoncées ci-après :
- les organes expansibles sont pivotants selon l'axe longitudinal du dispositif pour tordre la partie intermédiaire à un degré ajustable selon leur position angulaire relative.
- il comprend des moyens de positionnement de la zone de striction maximale.
- les moyens de positionnement comprennent au moins une liaison ponctuelle de deux zones de la paroi de la partie intermédiaire.
- il comporte deux liaisons ponctuelles à un même niveau de la longueur de la partie intermédiaire.
- il comprend un organe tubulaire creux placé entre les deux liaisons ponctuelles pour définir une lumière résiduelle.
- l'organe tubulaire comporte, dans son volume intérieur, une valve anti-retour.
- les moyens de positionnement comprennent des plis sur la surface de la partie intermédiaire, les plis étant concourants vers la zone de striction maximale.
- la partie intermédiaire est tubulaire et porte deux paires de plis orientées chacune selon un arc diagonal d'une moitié de la partie intermédiaire vue en coupe longitudinale, les plis de chaque paire étant formés de part et d'autre de la zone de striction maximale et étant d'épaisseur croissante dans sa direction.
- il comporte deux autres paires de plis de forme similaire aux deux premières et orientées suivant les deux arcs diagonaux complémentaires.
- les plis ont une forme semi-hélicoïdale.

L'invention concerne également un appareil de mise en place d'un dispositif d'occlusion vasculaire comportant deux organes expansibles creux pour sa fixation par appui sur deux portions de la paroi du vaisseau et une partie intermédiaire, creuse, déformable en torsion à un degré ajustable selon la position angulaire relative des deux organes expansibles, apte à être utilisé pour la mise en place d'un dispositif selon l'invention, caractérisé par le fait qu'il comporte :
- deux gaines cylindriques, creuses, l'une extérieure recevant le premier organe expansible et au moins une portion de la partie intermédiaire, l'autre intérieure et apte à une translation ainsi qu'à une rotation angulaire dans la gaine extérieure, apte à exercer une poussée sur le premier organe expansible, et recevant le deuxième organe expansible,
- un piston coulissant dans la gaine intérieure et apte à exercer une poussée sur l'extrémité libre du deuxième organe expansible.

Les dessins ci-joints sont donnés à titre d'exemples indicatifs et non limitatifs. Ils représentent un mode de réalisation préféré selon l'invention. Ils permettront de comprendre aisément l'invention.

Les figures 1 et 2 présentent des étapes d'utilisation du dispositif d'occlusion selon l'invention ainsi que les phases d'utilisation de l'appareil de mise en place.

La figure 3 illustre un exemple de réalisation du dispositif d'occlusion une fois implanté dans un vaisseau.

La figure 4 illustre la structure générale du dispositif d'occlusion selon l'invention dans un premier mode de réalisation et la figure 5 en schématise une configuration avec torsion de la partie intermédiaire.

La figure 6 illustre un deuxième mode de réalisation du dispositif d'occlusion vasculaire également visible en coupe selon la ligne A-A en figure 7.

Les figures 8 et 9 illustrent respectivement une vue longitudinale et une vue en coupe selon la ligne B-B d'un dispositif de torsion selon le mode de réalisation de la figure 6 dans une configuration où la partie intermédiaire est déformée en torsion.

Les figures 10 et 11 illustrent respectivement une vue longitudinale et une vue en coupe selon la ligne C-C du dispositif d'occlusion selon l'invention dans un mode de réalisation où un organe tubulaire est présent.

Les figures 12 à 16 illustrent d'autres modes de réalisation de l'invention dans lesquelles des plis sont constitués sur la surface de la partie intermédiaire.

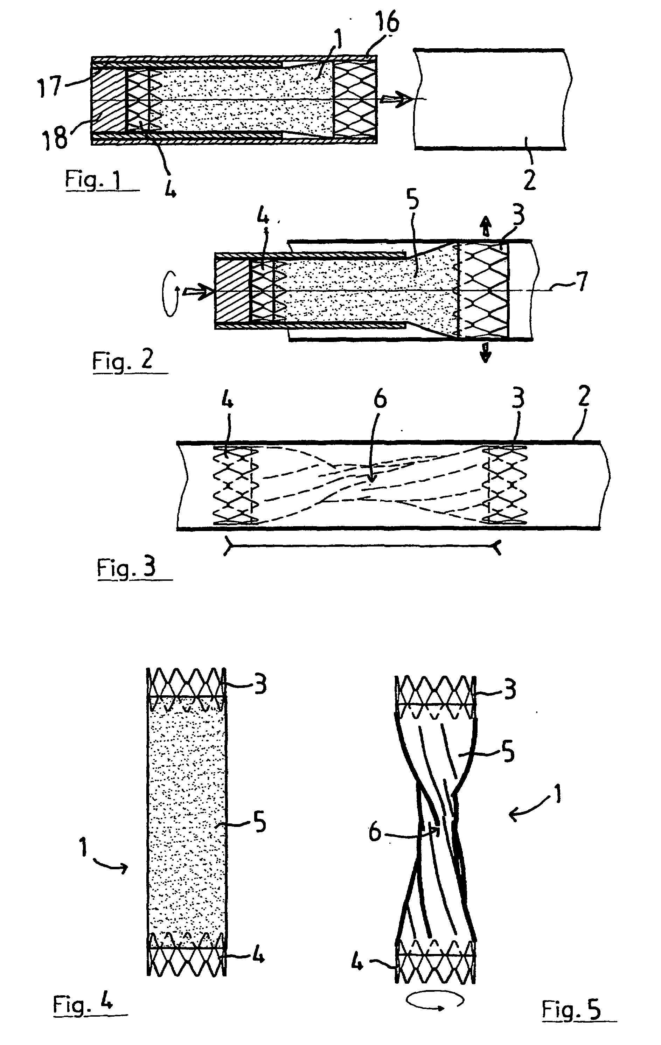

La figure 4 schématise un premier mode de réalisation du dispositif d'occlusion 1 selon l'invention.

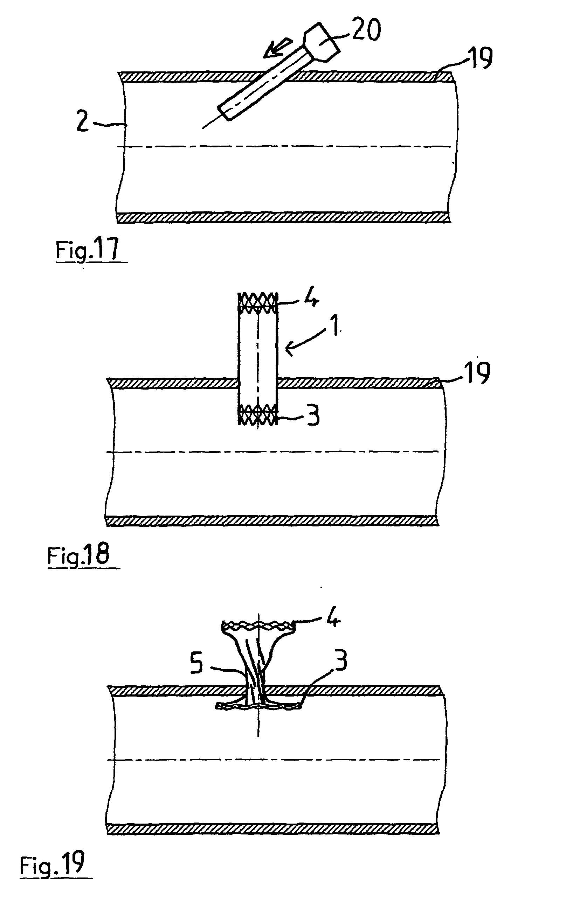

Les figures 17, 18 et 19 illustrent une application de l'invention à une occlusion pariétale.

Ce dispositif comprend deux organes expansibles 3, 4 visibles à la figure 4 aux deux extrémités du dispositif.

Les organes expansibles 3, 4 ont pour fonction d'assurer la fixation du dispositif dans le vaisseau 2 par appui sur deux portions de sa paroi interne.

A titre préféré mais non limitatif, les deux organes expansibles 3, 4 peuvent être constitués par des armatures de type stent auto expansibles à mémoire de forme ou encore déformables par inflation d'un ballon.

Entre les deux organes expansibles creux 3, 4 est présente une partie intermédiaire 5 également visible en figure 4.

La partie intermédiaire 5 est creuse et déformable en torsion.

Elle peut par exemple être constituée en un textile approprié.

De façon préférée, l'ensemble du dispositif ainsi réalisé a une forme sensiblement tubulaire creuse.

Tel qu'indiqué précédemment, la partie intermédiaire 5 est déformable en torsion.

Un exemple de cette déformation est présenté en figure 5.

Comme schématisé par des flèches, la déformation en torsion peut être produite par un déplacement angulaire relatif des organes expansibles 3, 4.

Il s'agira par exemple d'un pivot induit au niveau de l'armature de l'organe expansible 4 par rapport à l'organe expansible 3 qui restera lui-même fixe.

La déformation en torsion de la partie intermédiaire 5 conduit à la production d'une zone de striction maximale 6 qui, dans le cadre de la figure 5, est positionnée sensiblement à la moitié de la longueur de la partie intermédiaire 5.

La zone de striction maximale 6 est, suivant le degré de torsion, le lieu d'une occlusion partielle ou totale.

Ainsi, par exemple, au-delà d'un décalage angulaire des organes expansibles 3, 4 supérieurs à une limite préfixée (par exemple entre 180° et 360°), l'occlusion sera totale.

En deçà de cette valeur, l'occlusion sera partielle.

Il est utile de pouvoir positionner de façon précise la zone de striction maximale 6.

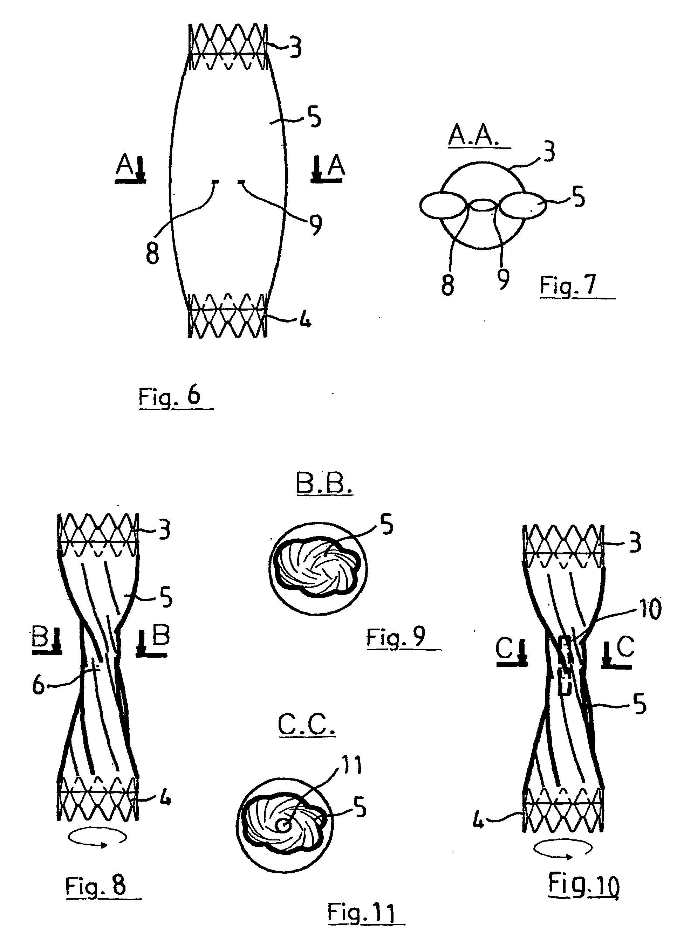

A cet effet, les figures 6 et 7 illustrent un deuxième mode de réalisation du dispositif d'occlusion 1.

Sur cette figure, des moyens de positionnement de la zone de striction maximale 6 sont visibles sous forme de deux liaisons ponctuelles 8, 9.

A tire d'exemple préféré, les liaisons ponctuelles 8, 9 sont des sutures réalisées sur la partie intermédiaire initialement constituée par un élément tubulaire creux.

On formera avantageusement deux liaisons ponctuelles 8, 9 situées au même niveau de la longueur de la partie intermédiaire 5 et positionnées de part et d'autre de l'axe longitudinal 7 de symétrie de la partie intermédiaire 5 tubulaire.

On remarque aux figures 8 et 9 que la torsion de la partie intermédiaire 5 est ainsi précisément orientée par les liaisons ponctuelles 8, 9 et que la zone de striction maximale 6 est parfaitement située.

Par ailleurs, dans un mode particulier de réalisation, les liaisons ponctuelles 8, 9 permettent de constituer une réservation apte à recevoir un organe tubulaire 10.

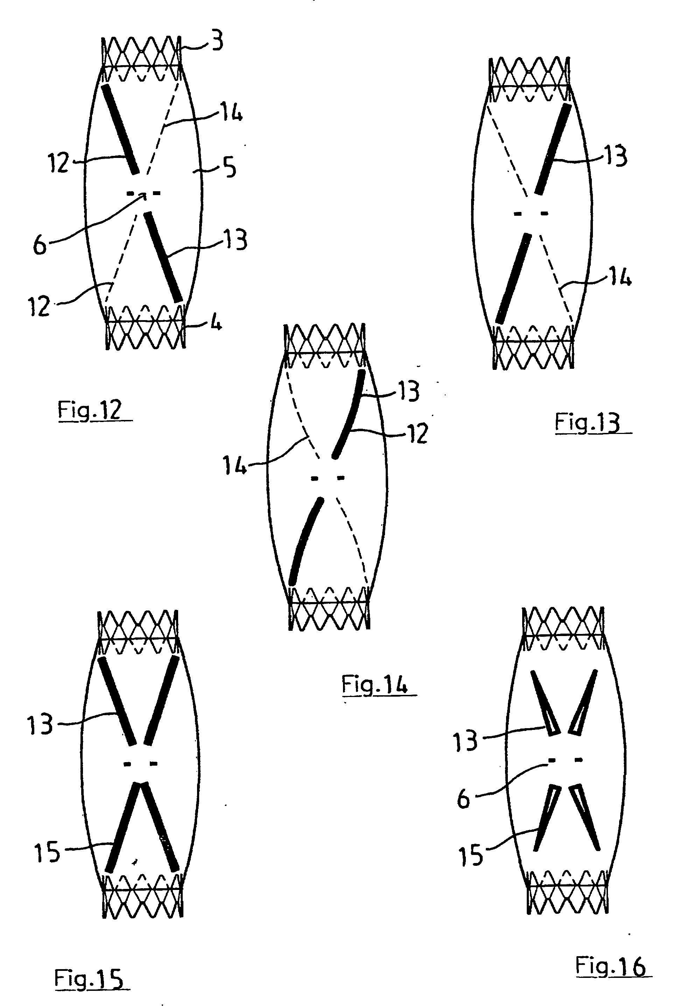

Celui-ci est placé entre les deux liaisons ponctuelles 8, 9 et définit une lumière résiduelle 11 représentée en figure 11.

On peut ainsi à coup sûr réaliser une occlusion partielle et limiter le passage du flux sanguin à une section bien définie.

Par ailleurs, pour le traitement de certaines pathologies, l'organe tubulaire 10 peut être le lieu d'implantation d'une valve anti-retour.

Ainsi, le dispositif d'occlusion ici présenté assurera une occlusion complète dans un sens et une occlusion partielle dans l'autre sens du flux sanguin.

Comme indiqué précédemment, il est précieux de pouvoir positionner aussi précisément que possible la zone striction maximale 6.

En complément ou en remplacement des liaisons ponctuelles 8, 9, on pourra à ce sujet constituer d'autres moyens de positionnement.

Ainsi, des plis 12 formés à la surface extérieure ou intérieure de la partie intermédiaire 5 peuvent assurer le positionnement et favorisent une première orientation de la torsion.

Les figures 12 à 16 illustrent successivement différentes variantes de réalisation et de configuration des plis 12.

De façon préférée, les plis sont formés sur la surface extérieure de la partie intermédiaire 5 constituée par un élément textile tubulaire creux.

Les plis sont concourants vers la zone de striction maximale 6 tel que cela ressort des figures 12 à 16.

Selon la figure 12, on forme deux paires 13, 14 de plis 12.

Une paire 13 est située sur une moitié de la partie intermédiaire 5, l'autre paire 14 est située sur l'autre moitié de la partie intermédiaire 5.

On entend par moitié la portion de la partie intermédiaire qui, vue en coupe longitudinale, est située d'un côté du plan de coupe.

Dans cette configuration, les paires 13, 14 sont orientées suivant deux arcs diagonaux de la partie intermédiaire 5 dans la configuration représentée à la figure 12.

Chaque arc diagonal porte deux plis 12 situés de part et d'autre de la zone de striction maximale 6 à former.

La figure 13 montre que l'on peut former des paires 13, 14 de plis situées suivant deux autres arcs diagonaux.

Dans le cas de la figure 14, les plis 12 ont une forme semi-hélicoïdale.

A la figure 15, on a représenté un mode de réalisation dans lequel outre les deux paires 13 ou 14 de plis 12, on constitue des paires complémentaires 15 permettant, sur chaque moitié de la partie intermédiaire 5, de constituer deux paires de plis.

Enfin, la figure 7 montre une autre variante de réalisation des plis 12 en ce sens que chaque pli 12 a une épaisseur croissante en direction de la zone de striction maximale 6.

Un pli sensiblement de forme triangulaire est donc réalisé dans ce mode de réalisation.

On comprend aisément que la formation des plis 12 facilite la déformation en torsion de la partie intermédiaire 5 et fixe de façon précise le lieu de la zone de striction maximale 6.

Le dispositif d'occlusion 1 ainsi présenté pourra être mis en place au moyen de l'appareil décrit ci-après qui fait parti intégrante de la présente invention.

En se référant à la figure 1, on voit que le dispositif d'occlusion 1 est intégré dans un appareil comprenant deux gaines l'une extérieure 16, l'autre intérieure 17.

Le dispositif d'occlusion 1 est situé de telle sorte que l'un des organes expansibles 3 est maintenu en position comprimée par l'une des gaines, par exemple, la gaine extérieure 16.

L'autre organe expansible 4 est maintenu en position comprimée par la gaine intérieure 17.

La partie intermédiaire 5 s'étend entre les deux organes expansibles 3, 4 et est située partiellement dans la gaine intérieure 17 et dans la gaine extérieure 16.

Les gaines 16, 17 sont bien entendu adaptées en longueur et en diamètre aux dimensions du dispositif d'occlusion 1.

Par ailleurs, leurs diamètres respectifs sont choisis de façon à coopérer dans la formation d'une liaison en pivot glissant.

A l'arrière de l'armature expansible 4 est constitué un piston 18 également visible en figure 1.

Ce piston s'applique dans la gaine intérieure 17 de façon à s'appuyer sur l'extrémité distale de l'armature de l'organe 4.

L'appareil ainsi présenté peut s'utiliser de la façon suivante.

On introduit l'appareil dans la partie du vaisseau 2 au niveau de laquelle l'occlusion est souhaitée.

En se référant à la figure 2, on constate qu'en esquivant la gaine extérieure 16 par rapport à la gaine intérieure 17, il est possible de libérer l'organe expansible 3.

Si cet organe 3 est du type autoexpansif, sa fixation sur la paroi interne du vaisseau sera produite d'emblée.

Il est également possible d'effectuer cette fixation par inflation d'un ballon à l'intérieur de l'organe expansible.

A cette étape de mise en place, le deuxième organe expansible 4 est libre en rotation et en déplacement axial par rapport au premier organe expansible 3.

La partie intermédiaire 5 subit des déformations notamment en torsion en fonction du mouvement imposé à l'organe expansible 4.

Ce mouvement est produit par le déplacement relatif de la gaine intérieure 17 par rapport au piston 18. Le piston 18 peut être du type communément appelé « pusher » dans le domaine considéré.

Ainsi, la rotation par le praticien de la gaine 17 entraîne la déformation en torsion de la partie intermédiaire 5 et le rapprochement corrélatif des deux organes expansibles 3, 4.

Une fois la position relative des organes expansifs 3, 4 souhaitée obtenue, on libère le deuxième organe expansible 4.

Cette libération s'effectue en esquivant la gaine intérieure 17 tout en maintenant appuyé le piston 18 sur l'organe expansible 4.

Après retrait de l'ensemble de l'appareil, on atteint une position d'implantation finale telle que présentée en figure 3.

Au cours de l'opération, il apparaît que le réglage du degré d'occlusion est parfaitement ajusté lors du mouvement opéré sur la gaine 17 notamment par la rotation qui lui est induite.

Bien entendu, l'appareil pourra comprendre différents repères notamment de déplacement angulaire longitudinal de la gaine 17 par rapport au piston 18 pour ajuster la torsion à produire sur la partie intermédiaire 5.

Le procédé d'utilisation qui fait parti également de l'invention peut être mis en oeuvre par l'appareil et le dispositif d'occlusion 1 qui ont été précédemment décrits.

Ces étapes de réalisation ressortent également notamment des figures 1 à 3.

Après la fixation de l'organe expansible 3, sur les parois internes du vaisseau 2, on pivote le deuxième organe expansible 4 par rapport au premier afin d'engendrer une torsion de la partie intermédiaire 5.

Il est ensuite possible de fixer le deuxième organe expansible 4 dans la position pivotée ajustée suivant le degré d'occlusion souhaité.

On notera qu'un guide central peut être incorporé dans l'appareil de mise en place sous forme d'un fil ou de cathéter au centre du dispositif à implanter, pour participer au déplacement du dispositif et d'éventuels accessoires de mise en place, dans le vaisseau.

Le guide central est, dans une variante, associé à un ballon qui peut être largable afin de rester à demeure au niveau de l'une des extrémités du dispositif implanté.

Les figures 17 à 19 montrent une application du dispositif d'occlusion 1 à l'occlusion pariétale.

Il peut arriver que le praticien ait à former un passage dans la paroi 19 du vaisseau 2, notamment lors de ponctions.

L'invention offre une possibilité de fermeture du passage.

La figure 17 montre l'introduction transpariétale d'un instrument chirurgical. Un passage dans la paroi 19 est ainsi formée.

En figure 18, un dispositif 1 est introduit au travers du passage dans la paroi 19. L'une 3 des extrémités constituées d'organes expansibles est alors insérée dans le volume intérieur du vaisseau 2. L'autre organe 4 est préservé à l'extérieur du vaisseau 2.

Par expansion, l'organe 3 s'applique sur la paroi interne du vaisseau 2 tandis que l'organe 4, après torsion, s'applique sur la paroi externe.

La figure 19 montre la déformation appliquée au dispositif pour cette application.

La partie intermédiaire 5 bouche le passage formé lors de l'intervention chirurgicale.

### REFERENCES

- 1.: Dispositif d'occlusion
- 2.: Vaisseau
- 3, 4.: Organes expansibles
- 5.: Partie intermédiaire
- 6.: Zone de striction maximale
- 7.: Axe longitudinal
- 8, 9.: Liaisons ponctuelles
- 10.: Organe tubulaire
- 11.: Lumière résiduelle
- 12.: Plis
- 13, 14.: Paires de plis
- 15.: Paire de plis complémentaire
- 16.: Gaine extérieure
- 17.: Gaine intérieure
- 18.: Piston
- 19.: Paroi
- 20.: Instrument

## Revendications

1. Dispositif d'occlusion (1) apte à être introduit dans un vaisseau (2) présentant deux organes expansibles creux (3, 4) pour sa fixation par appui sur deux portions de la paroi du vaisseau (2) et une partie intermédiaire (5), creuse, **caractérisé par le fait que**
la partie intermédiaire(s) est déformable en torsion à un degré ajustable selon la position angulaire relative des deux organes expansibles (3,4) pour créer une zone de striction maximale (6) définissant le degré d'occlusion.

2. Dispositif d'occlusion (1) selon la revendication 1 **caractérisé par le fait**
**que** les organes expansibles (3, 4) sont pivotants selon l'axe longitudinal (7) du dispositif (1) pour tordre la partie intermédiaire (5) à un degré ajustable selon leur position angulaire relative.

3. Dispositif d'occlusion (1) selon la revendication 1 ou 2, **caractérisé par le fait**
**qu'**il comprend des moyens de positionnement de la zone de striction maximale (6).

4. Dispositif d'occlusion (1) selon la revendication 3, **caractérisé par le fait**
**que** les moyens de positionnement comprennent au moins une liaison ponctuelle (8, 9) de deux zones de la paroi de la partie intermédiaire (5).

5. Dispositif d'occlusion (1) selon la revendication 4, **caractérisé par le fait**
**qu'**il comporte deux liaisons ponctuelles (8,9) à un même niveau de la longueur de la partie intermédiaire (5).

6. Dispositif d'occlusion (1) selon la revendication 5, **caractérisé par le fait**
**qu'**il comprend un organe tubulaire (10) creux placé entre les deux liaisons ponctuelles (8, 9) pour définir une lumière résiduelle (11).

7. Dispositif d'occlusion (1) selon la revendication 6, **caractérisé par le fait**
**que** l'organe tubulaire (10) comporte, dans son volume intérieur, une valve anti-retour.

8. Dispositif d'occlusion (1) selon l'une quelconque des revendications 3 à 7, **caractérisé par le fait**
**que** les moyens de positionnement comprennent des plis (12) sur la surface de la partie intermédiaire (5), les plis (12) étant concourants vers la zone de striction maximale (6).

9. Dispositif d'occlusion (1) selon la revendication 8, **caractérisé par le fait**
**que** la partie intermédiaire (5) est tubulaire et porte deux paires (13, 14) de plis orientées chacune selon un arc diagonal d'une moitié de la partie intermédiaire (5) vue en coupe longitudinale, les plis (12) de chaque paire (13, 14) étant formés de part et d'autre de la zone de striction maximale (6) et étant d'épaisseur croissante dans sa direction.

10. Dispositif d'occlusion (1) selon la revendication 9, **caractérisé par le fait**
**qu'**il comporte deux autres paires (15) de plis (12) de forme similaire aux deux premières et orientées suivant les deux arcs diagonaux complémentaires.

11. Dispositif d'occlusion (1) selon la revendication 9 ou 10 **caractérisé par le fait**
**que** les plis (12) ont une forme semi-hélicoïdale.

12. Appareil de mise en place d'un dispositif d'occlusion vasculaire comportant deux organes expansibles creux (3, 4) pour sa fixation par appui sur deux portions de la paroi du vaisseau (2) et une partie intermédiaire (5), creuse, déformable en torsion à un degré ajustable selon la position angulaire relative des deux organes expansibles (3, 4), apte à être utilisé pour la mise en place d'un dispositif (1) selon l'une quelconque des revendications 1 à 11, **caractérisé par le fait**
**qu'**il comporte :
- deux gaines cylindriques, creuses, l'une (16) extérieure apte à recevoir le premier organe expansible (3) et au moins une portion de la partie intermédiaire (5), l'autre (17) intérieure et apte à une translation ainsi qu'à une rotation angulaire dans la gaine extérieure (16), apte à exercer une poussée sur le premier organe expansible (3), et recevant le deuxième organe expansible (4),
- un piston (18) coulissant dans la gaine intérieure (17) et apte à exercer une poussée sur l'extrémité libre du deuxième organe expansible (4).

## Patentansprüche

1. Verschlussvorrichtung (1) zur Einführung in ein Blutgefäss (2) mit 2 hohlen, erweiterbaren Organen (3, 4) die durch Druck auf tzwei Abschnitten der Blutgefässwand (2) befestigt werden und einem hohlen Mittelabschnitt (5), **gekennzeichnet dadurch,**
**dass** der Mittelabschnitt bis zu einem bestimmten Maß verwunden werden kann, wobei das Maß von dem Winkel abhängt, den er mit den beiden erweiterbaren Organen (3, 4) bildet, um so eine Zone mit maximaler Querschnittsverminderung (6) zu erzeugen, die den Grad des Verschlusses bestimmt.

2. Verschlussvorrichtung (1) gemäß Anspruch 1, **gekennzeichnet dadurch, dass** die erweiterbaren Organe (3, 4) schwenkbar auf der Längsachse (7) der Vorrichtung (1) angeordnet sind, um den Mittelabschnitt (5) entsprechend ihrer relativen Winkelstellung auf ein einstellbares Maß zu verwinden.

3. Verschlussvorrichtung (1) gemäß Anspruch 1 oder 2, **gekennzeichnet dadurch, dass** sie Mittel zur Positionierung der Zone mit maximaler Querschnittsverminderung (6) aufweist.

4. Verschlussvorrichtung (1) gemäß Anspruch 3, **gekennzeichnet dadurch, dass** diese Positionierungsmittel mindestens eine punktförmige Verbindung (8, 9) von zwei Zonen der Wand des Mittelabschnitts (5) aufweisen.

5. Verschlussvorrichtung (1) gemäss Anspruch 4, **gekennzeichnet dadurch, dass** sie zwei auf dem Mittelabschnitt (5) in Längsrichtung auf gleicher Ebene angeordnete punktförmige Verbindungen (8, 9) besitzt.

6. Verschlussvorrichtung (1) gemäß Anspruch 5, **gekennzeichnet dadurch, dass** sie ein tubusartiges, hohles Organ (10) besitzt, das zwischen den beiden punktförmigen Verbindungen (8, 9) angeordnet ist, um einen offen verbleibenden Mindestquerschnitt (11) zu festzulegen.

7. Verschlussvorrichtung (1) gemäß Anspruch 6, **gekennzeichnet dadurch, dass** im Innenraum des tubusförmigen Organs (10) eine Rückschlagklappe vorhanden ist.

8. Verschlussvorrichtung (1) gemäß einem der Ansprüche 3 bis 7, **gekennzeichnet dadurch,**
**dass** die Mittel zur Positionierung Falten (12) an der Oberfläche des Mittelabschnitts (5) besitzen, und diese Falten (12) in Richtung auf die Zone der maximalen Querschnittsverringerung (6) zusammenlaufen.

9. Verschlussvorrichtung (1) gemäß Anspruch 8, **gekennzeichnet dadurch, dass** der Mittelabschnitt (5) tubusförmig ist und zwei Faltenpaare (13, 14) besitzt, von denen jedes auf einer Hälfte des Mittelabschnitts (5) im Längsschnitt gesehen in Richtung eines diagonal liegenden Bogens verläuft, wobei die Falten (12) jedes Faltenpaares (13, 14) auf jeder Seite der Zone mit maximaler Querschnittsverringerung (6) ausgebildet sind und ihre Dicke in ihrer Richtung zunimmt.

10. Verschlussvorrichtung (1) gemäß Anspruch 9, **gekennzeichnet dadurch, dass** sie zwei weitere Paare (15) von Falten (12) mit ähnlicher Form wie die beiden ersten besitzt, die in Richtung der komplementären diagonalen Bögen verlaufen.

11. Verschtussvorrichtung (1) gemäß Anspruch 9 oder 10, **gekennzeichnet dadurch,**
**dass** die Falten (12) eine halb schraubenartige Form besitzen.

12. Gerät zur Platzierung der Verschlussvorrichtung für Blutgefässe mit zwei erweiterbaren hohlen Organen (3, 4), die durch Druck auf zwei Abschnitte der Blutgefässwand (2) befestigt werden, und mit einem hohlen Mittelabschnitt, der bis zu einem bestimmten Ausmaß verwunden werden kann, wobei das Ausmaß von dem relativen Winkel der beiden erweiterbaren Organe (3, 4) abhängt, wobei das zur Platzierung einer Vorrichtung (1) gemäß einem der Ansprüche 1 bis 11 geeignete Gerät **gekennzeichnet ist dadurch**,
dass es folgende Elemente besitzt:
- zwei hohle zylindrische Hüllrohre, von denen das außenliegende (16), zur Aufnahme des erweiterbaren Organs (3) und mindestens eines Teils des Zwischenabschnitts (5) und das andere in diesem außenliegenden Rohr (16) eine Translations- und Rotationsbewegung ausführen und so auf das erste erweiterbare Organ (3) einen Druck ausüben kann und außerdem das zweite erweiterbare Organ (4) aufnehmen kann.
- einen Kolben (18), der in dem inneren Rohr (17) gleitet und auf das Ende des zweiten erweiterbaren Organs (4) einen Druck ausüben kann.

## Claims

1. Occlusion device (1) adapted to be introduced into a vessel (2) and comprising two hollow expansible members (3, 4) for its securement by bearing on two portions of the wall of the vessel (2) and an hollow middle section (5),
**characterised in that**
the middle section is deformable in torsion to an adjustable degree according to the relative angular position of the two expansible members (3, 4) to create a zone of maximum constriction (6) defining the degree of occlusion.

2. Occlusion device (1) according to claim 1 **characterized in that**
the expansible members (3, 4) pivot on longitudinal axis (7) of the device (1) to twist middle section (5) to a degree that is adjustable according to their relative angular position.

3. Occlusion device (1) according to claim 1 or 2, **characterized in that**
it includes means for positioning the zone of maximum constriction (6).

4. Occlusion device (1) according to claim 3, **characterized in that**,
the positioning features include at least one punctual connection (8, 9) of two zones of the wall of the middle section (5).

5. Occlusion device (1) according to claim 4, **characterized in that**
it includes two punctual connexions (8, 9) at a same level along the length of the middle section (5).

6. Occlusion device (1) according to claim 5, **characterized in that**
it includes a hollow tubular member (10) placed between two punctual connexions (8, 9) in order to define a residual opening (11).

7. Occlusion device (1) according to claim 6, **characterized in that**
tubular member (10) comprises in its internal volume, a non-return valve.

8. Occlusion device (1) according to any one of claims 3 to 7, **characterized in that**
the positioning means include folds (12) on the surface of middle section (5), folds (12) being convergent towards the zone of maximum constriction (6).

9. Occlusion device (1) according to claim 8, **characterized in that**
middle section (5) is tubular and has two pairs (13, 14) of folds directed each one along a diagonal arc of one half of the middle section (5) viewed in longitudinal cross-section, folds (12) of each pair (13, 14) being formed on opposite sides of the zone of maximum constriction (6) and being of a thickness increasing in its direction.

10. Occlusion device (1) according to claim 9, **characterized in that**
it includes two other pairs (15) of folds (12) of similar shape to the first two and oriented according to the two complementary diagonal arcs.

11. Occlusion device (1) according to claim 9 or 10, **characterized in that**
folds (12) have a semi-helicoidal shape.

12. Apparatus for emplacing a vascular occlusion device comprising two hollow expansible members (3, 4) for its securement by bearing against two portions of the wall of the vessel (2) and an hollow middle section (5), deformable in torsion to a degree that is adjustable according to the relative angular position of two expansible members (3, 4), suitable for use fir the emplacement of a device (1) according to any of claims 1 to 11, **characterized in that**
it includes:
- two hollow cylindrical sleeves, an external sleeve (16) receiving the first expansible member (3) and at least one section of the middle section (5), the other internal hollow cylindrical sleeve (17) movable by pivotal sliding in the external sleeve (16), and being adapted to exert a pressure on first expansible member (3), and receiving second expansible member (4),
- a piston (18) sliding in internal sleeve (17) and adapted to exert a pressure on the free end of the second expansible member 0(4).
